# EUROPEAN PATENT APPLICATION

(11) **EP 4 649 909 A1**
(43) Date of publication of application: **19.11.2025**
(21) Application number: 23916357.9
(22) Date of filing: 21.02.2023
(51) Int. Cl.: A61B 34/20, A61B 17/70, A61B 90/00, A61B 8/06, A61B 8/08, A61B 17/00

(54) **SCREW SURGICAL DEVICE AND METHOD**

(30) Priority: 11.01.2023 KR 20230003926
(71) Applicant: The Catholic University Of Korea Industry-Academic Cooperation Foundation, Seoul 06591 (KR)
(72) Inventor: HONG, Jae Taek, Seoul 03309 (KR)
(74) Representative: Bryn Aarflot AS
(86) International application number: PCT/KR2023/002453
(87) International publication number: WO 2024/150872

(57) **Abstract**

The present disclosure relates to a screw surgical device and method and can provide a screw surgical device and method, which use Doppler ultrasound using surgery in which pedicle screws are used, so as to sense the vertebral artery, and thus can reduce risk of the vertebral artery damage and amount of radiation exposure. Particularly, the present disclosure can provide the screw surgical device and method, which use Doppler ultrasound using surgery so as to monitor blood flow information about arterial blood adjacent to an insertion path of a pedicle screw, and thus can improve accuracy of pedicle screw fixation.

## Description

### [Technical Field]

Embodiments provide a screw surgical device and method.

### [Background Art]

The vertebral column forms the central axis of the body's torso and includes the spine composed of 7 cervical vertebrae, 12 thoracic vertebrae, and 5 lumbar vertebrae and an intervertebral disc which is called an intervertebral disc or a disc and is a disc-shaped cartilaginous structure which connects the spine to the spine. Through this, the vertebral column can play a role in maintaining and supporting a posture according to physical exercise of a person. Here, the vertebral column can gradually become deformed due to various causes such as aging, excessive exercise, or habitual bad posture, which can cause various neurological abnormalities. Particularly, diseases associated with the vertebral column may include vertebral column scoliosis, lordosis (kyphosis), vertebral column dislocation, fracture, and vertebral column separation. In particularly, in recent years, as the aging population increases, the frequency of being diagnosed with vertebral column-related diseases such as degenerative vertebral column stenosis, vertebral column anterior displacement, and thoracolumbar fractures is also increasing. A method for treating such vertebral column diseases is to perform a vertebral column fixation technique which physically fixes the bone causing instability and connects two or more adjacent vertebral column objects into one.

Among such vertebral column disease fixation techniques, surgical instruments used in a cervical spine fixation technique may include pedicle screws. Fixation using pedicle screws in cervical spine disease is receiving attention because the fixation is biomechanically stronger than external mass screw fixation, shortens the bone union period, and has the effect of correcting deformity. Particularly, pedicle screws can be interconnected through connection rods while embedded in the vertebral column object to maintain an interval between adjacent bones. A vertebral column fixation technique in which pedicle screws are used may be essential for decompression and fusion of the vertebral column. Here, there is a problem in that, since the vertebral column fixation technique in which pedicle screws are used depends on the clinical knowledge and experience of the vertebral column surgeon in selecting the pedicle screws, in the case of an inexperienced surgeon, there is a high risk of causing serious complications such as cervical spine nerve damage, screw fracture with rupture of the vertebral artery or the like, and an abnormal position of a screw which can occur due to incorrect screw insertion. Furthermore, if a radiographic device such as an X-ray is used in the process of checking the screw insertion path during surgery, there is also the problem in that the surgery will take a long time and there is a risk of secondary damage to the patient and the surgeon which can occur due to radiation.

Therefore, a screw surgical device and method which use Doppler ultrasound during surgery in which pedicle screws are used to sense the vertebral artery, and thus can reduce a risk of vertebral artery damage and an amount of radiation exposure due to use a radiological image during surgery are required.

### [Summary of Invention]

### [Technical Problem]

With such background, embodiments provide a screw surgical device and method which use Doppler ultrasound during surgery in which pedicle screws are used to sense the vertebral artery, and thus can reduce a risk of vertebral artery damage and an amount of radiation exposure.

### [Solution to Problem]

In order to achieve the above-described purpose, in an aspect, according to an example embodiment, a screw surgical device which includes: an instrument part which includes a gear shift and a taper which are inserted from a target patient's skin to a predetermined point in a pedicle to form an insertion path and a screw which is inserted and fixed into a spine through the insertion path; a sensor part which monitors blood flow information about blood flow passing through an artery adjacent to the insertion path using an ultrasound probe installed at one end of the instrument part; and an information providing part which senses a vertebral artery (Vertebral artery, VA) on the basis of the blood flow information and provides guide information about the insertion path is provided.

In another aspect, according to , an example embodiment, a screw surgery method which includes: inserting an instrument part which includes a gear shift and a taper which are inserted from a target patient's skin to a predetermined point in a pedicle to form an insertion path and a screw which is inserted and fixed into a spine through the insertion path; monitoring blood flow information about a blood flow passing through an artery adjacent to the insertion path using an ultrasound probe installed at one end of the instrument part; and sensing a vertebral artery (Vertebral artery, VA) on the basis of the blood flow information and providing guide information about the insertion path is provided.

### [Advantageous Effects of Invention]

According to example embodiments, the example embodiments can provide a screw surgical device and method which use Doppler ultrasound during surgery in which pedicle screws are used to sense the vertebral artery, and thus can reduce a risk of vertebral artery damage and an amount of radiation exposure.

### [Brief Description of Drawings]

FIG. 1 is a drawing showing a system to which a screw surgical device according to an example embodiment of the present disclosure may be applied.
FIG. 2 is a drawing showing a configuration of a screw surgical device according to an example embodiment of the present disclosure.
FIG. 3 is a drawing for explaining an instrument part of a screw surgical device according to an example embodiment of the present disclosure.
FIG. 4 is a drawing for explaining an example of a Doppler ultrasound probe sensing the vertebral artery according to an example embodiment of the present disclosure.
FIGS. 5 and 6 are drawings showing an example of a screw surgical device monitoring arterial blood according to an example embodiment of the present disclosure.
FIG. 7 is a flowchart for describing a screw surgery method according to an example embodiment of the present disclosure.

### [Description of Embodiments]

The present disclosure relates to a screw surgical device and method.

Some example embodiments of the present disclosure are described in detail below with reference to the illustrative drawings. When the constituent elements in each of the drawings are denoted by reference numerals, it should be noted that the same constituent elements are denoted by the same reference numerals as much as possible even if they are shown in different drawings. Furthermore, when explaining the present disclosure, if it is determined that a detailed description of the relevant notice structure or function may obscure the main point of the present disclosure, such detailed description will be omitted.

Also, in describing the constituent elements of the present disclosure, the terms first, second, A, B, (a), (b), and the like may be used. Such terms are only intended to distinguish one constituent element from another constituent element and do not limit the nature, the order, or the sequence of the constituent element. When a constituent element is described as being "connected," "joined," or "connected" to another constituent element, it should be understood that the constituent element may be directly connected or connected to the other constituent element, but that between each constituent element, another constituent element may also be "connected," "joined," or "connected."

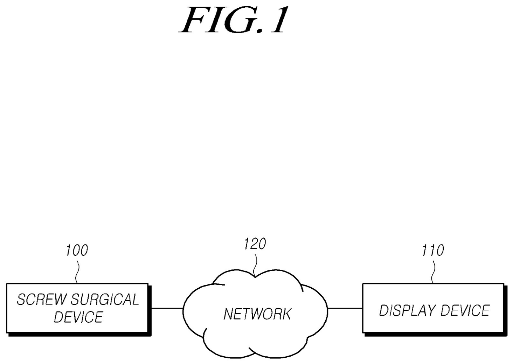

FIG. 1 is a drawing showing a system to which a screw surgical device according to an example embodiment of the present disclosure may be applied.

Referring to FIG. 1, a system to which a screw surgical device 100 according to an example embodiment of the present disclosure may be applied may be implemented to include a network 120 and a display device 130. Furthermore, the screw surgical device 100 is a device which can perform a screw surgery method according to an example embodiment of the present disclosure and may be implemented as hardware such as an electronic device which can execute a program, software executed by a processor, or a combination thereof.

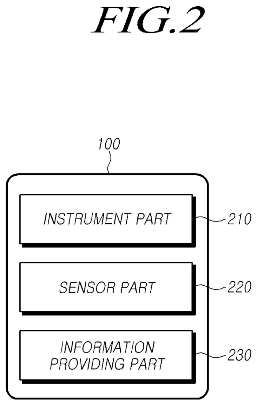

According to an example, the screw surgical device 100 may be a device which may be directly inserted into a surgical site corresponding to a target patient's body to perform surgery. The screw surgical device 100 includes an instrument part 210, a sensor part 220, and the information providing part 230 and a detailed description thereof will be described below with reference to FIG. 2.

As an example, the display device 130 can display guide information provided through blood flow information monitored during screw surgery. Furthermore, the display device 130 can provide, in an audible way, a visual way, or in both ways, alarm information generated in accordance with the proximity of the ultrasound probe and the vertebral artery as guide information. For example, the display device 130 may correspond to a computing device including a display screen. Particularly, a cathode-ray tube (CRT), a liquid crystal display (LCD), a plasma display panel (PDP), or a light emitting diode (LED) may be implemented as the display device 130, but are not necessarily limited thereto and various devices including a display panel may also be implemented as the display device 130. The display device 130 can be connected to the screw surgical device 100 through the network 120 and at least one of the display devices 130 may also be connected to the screw surgical device 100 simultaneously.

For example, the display device 130 may receive and display related data during surgery of the target patient such as ultrasound images, ultrasound signals, and Doppler signals of the target patient through the network 120. Furthermore, the display device 130 may also receive and display medical images captured using other medical devices such as computed tomography (CT), magnetic resonance imaging (MRI), and X-ray through the network 120. In addition, the display device 130 can perform data communication not only with a server or a medical device in the hospital, but also with a portable terminal of a doctor or a customer and may also display information about the target patient's diagnosis history or treatment schedule so that the information may be utilized.

On the other hand, as an example, the network 120 is a network which connects the screw surgical device 100 and the display device 130 and may be a closed network such as a local area network (LAN) or a wide area network (WAN), but may also be an open network such as the Internet. Here, the Internet refers to a worldwide open computer network structure which provides various services existing on a transmission control protocol/Internet protocol (TCP/IP) protocol and an upper layers thereof, that is, HyperText Transfer Protocol (HTTP), Telnet, a file transfer protocol (FTP), a domain name system (DNS), a simple mail transfer protocol (SMTP), a simple network management protocol (SNMP), a network file service (NFS), and a network information service (NIS).

The screw surgical device and method according to an example embodiment of the present disclosure briefly described above will be described in more detail below.

FIG. 2 is a drawing showing a configuration of a screw surgical device according to an example embodiment of the present disclosure.

Referring to FIG. 2, the screw surgical device 100 according to an example embodiment of the present disclosure may include the instrument part 210 which includes a gear shift and a taper which are inserted from the target patient's skin to a predetermined point in the pedicle to form an insertion path and a screw which is inserted and fixed into the spine through the insertion path, the sensor part 220 which monitors blood flow information about a blood flow passing through an artery adjacent to the insertion path using an ultrasound probe installed at one end of the instrument part, and the information providing part 230 which senses the vertebral artery (Vertebral artery, VA) on the basis of the blood flow information and provides guide information about the insertion path.

The instrument part 210 according to an example embodiment may include the gear shift, the taper, and the screw. For example, the instrument part 210 may include the gear shift and the taper which are inserted from the target patient's skin into a predetermined point in the pedicle to form an insertion path and the screw which is inserted and fixed into the spine through the insertion path. For example, the instrument part 210 may be installed so that each of the ultrasound probes is secured at one end of the gear shift and the taper in the insertion direction. Furthermore, the instrument part 210 may be installed so that a separate ultrasound probe may be inserted and removed through a receiving space formed inside the screw. Particularly, the gear shift and the taper may be instruments which form an insertion path so that the screw may approach a predetermined point in the pedicle that is a surgical target of the target patient. In addition, the screw may be an instrument which is fixed to the cervical spine from the C2 to C6 levels in which a V2 segment of the vertebral artery traverses. Here, the insertion path may be the shortest distance from the target patient's skin to a predetermined point for fixing the screw in the pedicle or a path formed for minimizing damage to the internal artery.

The sensor part 220 according to an example embodiment may monitor blood flow information using an ultrasound probe. As an example, the sensor part 220 may monitor blood flow information about a blood flow passing through an artery adjacent to the insertion path using an ultrasound probe installed at one end of the instrument part. For example, the sensor part 220 may monitor blood flow information by transmitting an ultrasound signal to a target object along an insertion path and receiving an ultrasound signal reflected from the target object. Furthermore, the sensor part 220 may monitor blood flow information using Doppler ultrasound signals. Here, the ultrasound probe may be a Doppler ultrasound probe with a 2 mm tip. Particularly, in the sensor part 220, the ultrasound probe installed at one end of the instrument part may monitor blood flow information using Doppler ultrasound signals along a wall of the insertion path. In addition, the sensor part 220 may monitor blood flow information which includes a blood flow waveform and a blood flow sound which appear due to changes in blood flow velocity in real time using a Doppler ultrasound signal.

The information providing part 230 according to an example embodiment may provide guide information about the insertion path during surgery. As an example, the information providing part 230 may sense the vertebral artery on the basis of the blood flow information and provide guide information about the insertion path. Furthermore, the information providing part 230 may generate alarm information in accordance with the proximity of the ultrasound probe and the vertebral artery on the basis of the blood flow information and provide the alarm information as guide information. Here, the proximity may change in real time in accordance with an insertion direction and an insertion depth of the ultrasound probe. For example, the information providing part 230 may sense that the ultrasound probe has come into contact with the vertebral artery if the blood flow waveform and the blood flow sound included in the blood flow information are outside of a preset normal range. Moreover, the information providing part 230 may generate alarm information if it is sensed that the ultrasound probe has come into contact with the vertebral artery and provide the alarm information as guide information for adjusting the insertion path. As another example, the information providing part 230 may calculate insertion depth information of the screw according to the insertion path on the basis of the blood flow information and provide the insertion depth information as guide information. Particularly, the information providing part 230 may sense the vertebral artery in real time during surgery and calculate insertion depth information of the screw on the basis of a point at which the ultrasound probe and the vertebral artery come into contact with each other. Furthermore, the information providing part 230 may provide the calculated insertion depth information of the screw as guide information for determining a length of the screw. Thus, the screw surgical device 100 senses the vertebral artery by monitoring blood flow information in real time during surgery, thereby more accurately adjusting the insertion path of the instrument part during surgery and determining an appropriate length of the screw. Therefore, the screw surgical device 100 can provide the effect of preventing damage to the vertebral artery due to screw disposition during surgery.

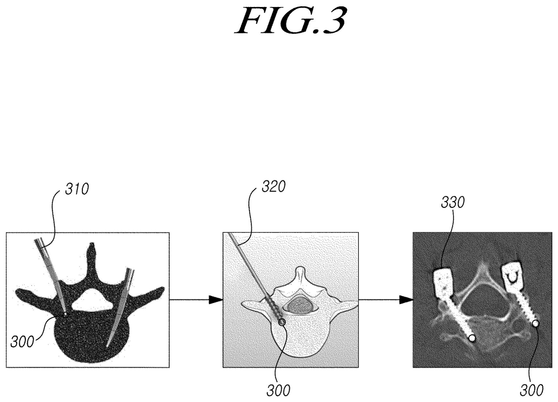

FIG. 3 is a drawing for explaining an instrument part of a screw surgical device according to an example embodiment of the present disclosure.

Referring to FIG. 3, a configuration of the instrument part 210 of the screw surgical device 100 according to an example embodiment of the present disclosure is explained. As an example, the instrument part 210 of the screw surgical device 100 may include a gear shift 310, a taper 320, and a screw 330 which are sequentially used for each step of surgery in which pedicle screws are used.

For example, the gear shift 310 may be an instrument which is first inserted to a predetermined point in the pedicle of a target patient to check the insertion depth of the screw 330. Particularly, the gear shift 310 is a pedicle finder or a drill with a displayed depth gauge which may form a hole having a diameter smaller than that of the screw 330 along the insertion path. Furthermore, the gear shift 310 may be installed so that a ultrasound probe 300 is fixed at a terminal of one end in the insertion direction. Thus, the screw surgical device 100 may check the insertion path in real time through the ultrasound probe 300 installed in the gear shift 310, and if the insertion path is incorrect, it may form a different insertion path.

As another example, the taper 320 may be an instrument inserted through a hole formed using the gear shift 310 to secure an insertion path for the screw 330. Particularly, the taper 320 may form a hole with a threaded shape on the inside diameter through the threads on the surface. Furthermore, the taper 320 may be installed so that the ultrasound probe 300 is fixed at a terminal of one end in the insertion direction. Here, the taper 320 may deviate from the insertion path due to a diameter difference between the taper 320 and the gear shift 310. Thus, the screw surgical device 100 may be formed while checking the insertion path in real time through the ultrasound probe 300 installed in the taper 320.

As still another example, the screw 330 may be an instrument which is inserted and fixed into the spine through the insertion path formed using the taper 320. That is to say, the screw 330 may be inserted and fixed into a sequentially formed insertion path. Particularly, the screw 330 may be installed so that the ultrasound probe 300 is inserted through the receiving space formed therein and may be removably installed if the screw 330 is fixed. Here, the receiving space formed inside the screw 330 has a shape in which an open tube is provided and may allow the ultrasound probe 300 to be inserted and removed.

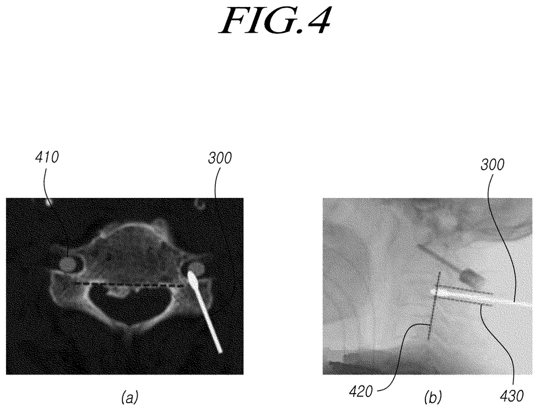

FIG. 4 is a drawing for explaining an example of a Doppler ultrasound probe sensing the vertebral artery according to an example embodiment of the present disclosure.

Referring to an axial CT scan of FIG. 4(a), an example of the ultrasound probe 300 sensing the vertebral artery during surgery according to an example embodiment of the present disclosure is explained. For example, the ultrasound probe 300 may be utilized to sense the vertebral artery 410 during insertion and placement of pedicle screws.

Referring to an X-ray image during surgery in FIG. 4(b), another example of the ultrasound probe 300 sensing the vertebral artery during surgery according to an example embodiment of the present disclosure is explained. As an example, the ultrasound probe 300 may be used for determining whether an insertion path 430 of a screw is appropriate during surgery. For example, the ultrasound probe 300 may monitor the direction and the depth of the insertion path 430 formed using the gear shift, the taper, and the screw. Therefore, if the ultrasound probe 300 senses an artery blood flow around a posterior spinal line 420, the insertion path 430 of the screw can be formed to deflect laterally and face the transverse foramen.

Also, the accuracy of screw surgery of the screw surgical device 100 according to an example embodiment of the present disclosure is shown in Table 1.

**[Table 1]**

| Screw Position | Total (n = 399) | Doppler (n = 186) | Control (n = 213) | *P* Value |
|---|---|---|---|---|
| 1 | 302 (75.7%) | 15B (84.9%) | 144 (67.6%) | |
| 2 | 61 (15.3%) | 24 (12.9%) | 37 (17.4%) | |
| 1+2 | 363 (91.0%) | 182 (97.8%) | 181 (85.0%) | <.01 |
| 3 | 28 (7.0%) | 3 (1.6%) | 25 (11.7%) | |
| 4 | 5 (1.3%) | 0 (0%) | 5 (2.3%) | |
| 5 | 3 (8%) | 1 (.5%) | 2 (.9%) | |
| 3+4+5 | 36 (9.0%) | 4 (2.2%) | 32 (15.0%) | <.01 |

For example, the screw surgical device 100 may insert pedicle screws using a Doppler ultrasound probe. In pedicle screws inserted in this way, Of 186 pedicle screws, 138 (74.2%) pedicle screws were be fixed at C2 and 48 (25.8%) pedicle screws were be fixed at C3 to C6. Furthermore, it was checked that the pedicle screws using the screw surgical device 100 were inserted into the correct position 97.8% of the time. That is to say, the screw surgical device 100 has a high rate of inserting pedicle screws into the correct position (Doppler 97.8%, Control 85.0%) and may provide the effect of not causing vertebral artery damage.

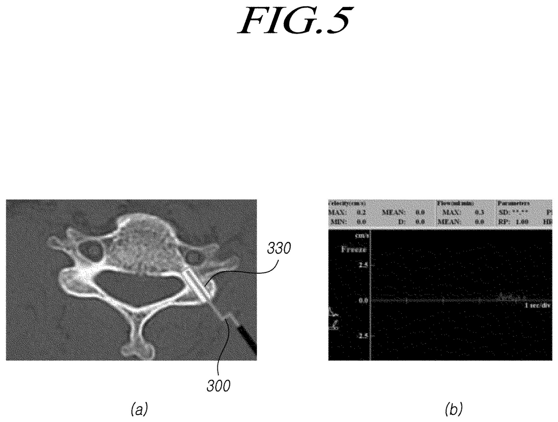

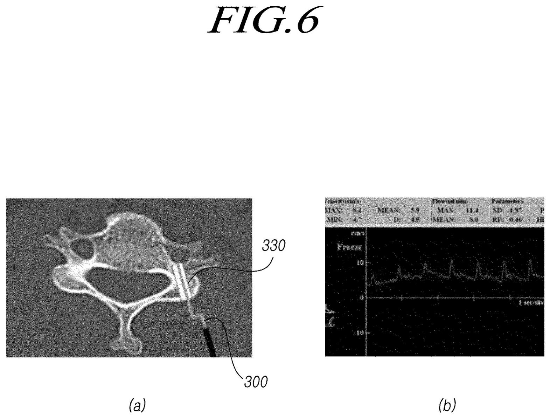

FIGS. 5 and 6 are drawings showing an example of a screw surgical device monitoring arterial blood according to an example embodiment of the present disclosure.

Referring to FIG. 5, an example embodiment of the present disclosure can explain an example of the screw surgical device 100 monitoring arterial blood using an ultrasound probe during surgery. FIG. 5(a) is an example of a case in which there is no artery blood flow on the insertion path of the ultrasound probe 300 inserted through the inside of the screw 330 and FIG. 5(b) may be an example of blood flow information monitored through the ultrasound probe 300. As an example, the screw surgical device 100 may form an optimal insertion path by monitoring arterial blood through the ultrasound probe 300 inserted into the screw 330. For example, the screw surgical device 100 may sense the vertebral artery and provide guide information about the insertion path while inserting and placing the screw 330 through the ultrasound probe 300 during surgery. Here, when there is no the vertebral artery on the insertion path from an entry point in which the ultrasound probe 300 is inserted, changes in the blood flow waveform and the blood flow sound included in the blood flow information may not appear. In this case, the screw surgical device 100 may not provide separate alarm information during surgery.

Referring to FIG. 6, another example in which the screw surgical device 100 according to an example embodiment of the present disclosure monitors arterial blood using an ultrasound probe during surgery is described. FIG. 6(a) is an example of a case in which there is artery blood flow on the insertion path of the ultrasound probe 300 inserted through the inside of the screw 330 and FIG. 6(b) is an example of blood flow information monitored through the ultrasound probe 300. For example, the screw surgical device 100 may sense that the vertebral artery is located close to the insertion path through the ultrasound probe 300 during surgery. That is to say, the screw surgical device 100 may sense whether there is a contact using changes in blood flow information which appear as the ultrasound probe 300 and the vertebral artery approach each other. Thus, the screw surgical device 100 may sense that the ultrasound probe 300 and the vertebral artery come into contact with each other if the blood flow waveform and the blood flow sound included in the monitored blood flow information are outside of a preset normal range. Here, although the normal range may be set to a case in which a blood flow waveform and a blood flow sound do not appear, the present disclosure is not limited to this as the normal range may change in accordance with the patient's physical information and treatment site. Here, the target patient's vertebral artery blood flow velocity measured through the ultrasound probe 300 during surgery may be within a range from 6.8 cm/s to 22 cm/s.

As another example, the screw surgical device 100 may generate alarm information in accordance with the proximity of the ultrasound probe to the vertebral artery on the basis of blood flow information and provide the alarm information as guide information. Particularly, the screw surgical device 100 may sense that the ultrasound probe 300 and the vertebral artery come into contact with each other if a change in the blood flow waveform and the blood flow sound included in the monitored blood flow information occurs and generate alarm information. Furthermore, the screw surgical device 100 may sense an inappropriate lateral deviation in the insertion path of the screw 330 during surgery on the basis of the alarm information fed back when the screw 330 is directed toward the transverse foramen. Here, the alarm information is generated through at least one of the following senses: auditory, visual, and tactile, but is not limited to these, as long as a user can perceive it. For example, it is possible to provide guide information in a form in which vibrations or a beeping sound is produced in the ultrasound probe 300 or an external display device 110 of the screw surgical device 100 or the insertion path and the proximity are visually displayed.

A screw surgery method which may be performed by the screw surgical device described with reference to FIGS. 1 to 6 will be described below.

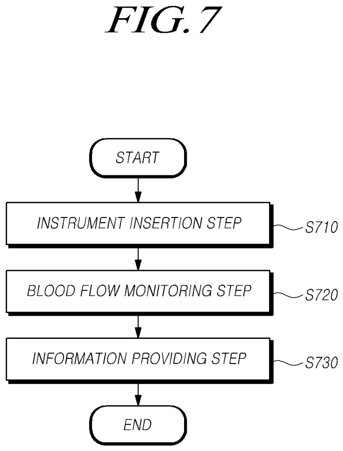

FIG. 7 is a flowchart for describing a screw surgery method according to an example embodiment of the present disclosure.

Referring to FIG. 7, a screw surgery method according to an example embodiment of the present disclosure may be a method for controlling an operation of a screw surgical device which includes the instrument part 210 which includes the gear shift and the taper which are inserted from the target patient's skin to a predetermined point in the pedicle to form an insertion path and the screw which is inserted and fixed into the spine through the insertion path, the sensor part 220 which monitors blood flow information about a blood flow passing through an artery adjacent to the insertion path using an ultrasound probe installed at one end of the instrument part, and the information providing part 230 which senses the vertebral artery (Vertebral artery, VA) on the basis of the blood flow information and provides guide information about an insertion path.

The screw surgery method according to an example embodiment may include an instrument insertion step (S710). As an example, the screw surgical device may insert an instrument part which includes the gear shift and the taper which are inserted from the target patient's skin to a predetermined point in the pedicle to form an insertion path and the screw which is inserted and fixed into the spine through the insertion path. For example, the gear shift and the taper may be installed so that each ultrasound probe is fixed at a terminal of one end in the insertion direction. Furthermore, the screw may be installed so that a separate ultrasound probe may be inserted and removed through the receiving space formed therein. Particularly, the gear shift and the taper may be instruments which form an insertion path so that the screw may approach a predetermined point in the pedicle that is a surgical target of the target patient. In addition, the screw may be an instrument which is fixed into the cervical spine from the C2 to C6 levels in which the V2 segment of the vertebral artery traverses. Here, the insertion path may have the shortest distance from the target patient's skin to a predetermined point in the pedicle or a path formed to minimize damage to the internal artery.

The screw surgery method according to an example embodiment may include a blood flow monitoring step (S720). As an example, the screw surgical device may monitor blood flow information about a blood flow passing through an artery adjacent to the insertion path using an ultrasound probe installed at one end of the instrument part. For example, the screw surgical device may monitor blood flow information by transmitting an ultrasound signal to a target object on the insertion path using an ultrasound probe and receiving an ultrasound signal reflected from the target object. Furthermore, the screw surgical device may monitor blood flow information using Doppler ultrasound signals. Here, the ultrasound probe may be a Doppler ultrasound probe with a 2 mm tip. Particularly, the screw surgical device may monitor blood flow information using Doppler ultrasound signals along the wall of the insertion path through an ultrasound probe installed at a terminal of one end of the instrument part. In addition, the screw surgical device may monitor, in real time, blood flow information which includes the blood flow waveform and the blood flow sound which appear due to changes in blood flow velocity using Doppler ultrasound signals.

The screw surgery method according to an example embodiment may include an information providing step (S730). As an example, the screw surgical device may sense the vertebral artery on the basis of the blood flow information and provide guide information about the insertion path. Furthermore, the screw surgical device may generate alarm information in accordance with the proximity of the ultrasound probe to the vertebral artery on the basis of the blood flow information and provide the alarm information as guide information. For example, the screw surgical device may sense that an ultrasound probe and the vertebral artery has come into contact with each other if the blood flow waveform and the blood flow sound included in the blood flow information are outside of a preset normal range. Furthermore, the screw surgical device may generate alarm information if it is sensed that the ultrasound probe comes into contact with the vertebral artery and provide the alarm information as guide information for adjusting the insertion path. As another example, the screw surgical device may calculate insertion depth information of the screw according to the insertion path on the basis of the blood flow information and provide the insertion depth information as guide information. Particularly, the screw surgical device may sense the vertebral artery in real time during surgery and calculate screw insertion depth information on the basis of the point of contact between the ultrasound probe and the vertebral artery. In addition, the screw surgical device may provide the generated screw insertion depth information as guide information for determining a length of the screw. Thus, the screw surgical device may sense the vertebral artery by monitoring blood flow information in real time during surgery, thereby more accurately adjusting the insertion path of the instrument part during surgery and determining an appropriate length of the screw. Therefore, the screw surgical device may provide the effect of preventing damage to the vertebral artery due to screw disposition during surgery.

As explained above, according to the present disclosure, it is possible to provide a screw surgical device and method which use Doppler ultrasound during surgery in which pedicle screws are used to sense the vertebral artery, and thus can reduce a risk of the vertebral artery damage and an amount of radiation exposure. Particularly, it is possible to provide a screw surgical device and method which use Doppler ultrasound during surgery to monitor blood flow information about arterial blood adjacent to an insertion path of a pedicle screw.

In the above, although all constituent elements constituting the example embodiment of the present disclosure have been described as being combined or operating as one, the present disclosure is not necessarily limited to this example embodiment. That is to say, within the scope of the purpose of the present disclosure, all of the constituent elements may optionally be combined to operate in one or more combinations. Furthermore, although all of the constituent elements may be implemented as independent hardware, some or all of the constituent elements may be selectively combined to implement a computer program having program modules which perform some or all of the functions of the combined hardware, and may be recorded on a computer-readable medium. The above computer-readable medium may include program commands, data files, data structures, and the like, alone or in combination. The program commands recorded on the medium may be those specially designed and configured for embodiment or may be known and available to those skilled in the art of computer software. Examples of computer-readable recording media include magnetic media such as hard discs, floppy discs, and magnetic tape, optical media such as compact disc read-only memories (CD-ROMs) and digital versatile discs (DVDs), magneto-optical media such as floptical disks, and hardware devices specifically configured to store and execute program instructions such as read-only memories (ROMs), read-access memories (RAMs), and flash memories. Examples of program instructions include machine language code such as program instructions produced by a compiler, as well as high-level language code which can be executed by a computer using an interpreter and the like. The above hardware devices may be configured to operate as one or more software modules to perform the operations of the embodiment, and vice versa.

Also, the terms "include," "comprise," or "have," as used herein, unless otherwise specifically stated, imply that the constituent element may be included, and thus should be interpreted as including other constituent elements rather than excluding other constituent elements. All terms, including technical or scientific terms, unless otherwise defined, have the same meaning as commonly understood by a person of ordinary skill in the art to which the present disclosure belongs. Commonly used terms such as terms defined in the dictionary should be interpreted consistent with their meaning in the context of the relevant technology and will not be interpreted in an idealized or overly formal sense unless expressly defined in the present disclosure.

The above explanation is only an example of the technical idea of the present disclosure and those with ordinary skill in the technical field to which the present disclosure belongs may make various modifications and variations without departing from the essential characteristics of the present disclosure. Therefore, the example embodiments disclosed in the present disclosure are intended to illustrate rather than limit the technical ideas of the present disclosure and the scope of the technical ideas of the present disclosure is not limited by these example embodiments. The scope of protection of the present disclosure should be interpreted by the claims below and all technical ideas within the equivalent scope should be interpreted as being included in the scope of protection of the present disclosure.

## Claims

1. A screw surgical device comprising:
an instrument part which includes a gear shift and a taper which are inserted from a target patient's skin to a predetermined point in a pedicle to form an insertion path and a screw which is inserted and fixed into a spine through the insertion path;
a sensor part which monitors blood flow information about blood flow passing through an artery adjacent to the insertion path using an ultrasound probe installed at one end of the instrument part; and
an information providing part which senses a vertebral artery (Vertebral artery, VA) on the basis of the blood flow information and provides guide information about the insertion path.

2. The screw surgical device of claim 1, wherein the instrument part is installed so that each ultrasound probe is fixed at a terminal of one end of the gear shift and the taper in an insertion direction.

3. The screw surgical device of claim 1, wherein the instrument part is installed so that a separate ultrasound probe is able to be inserted and removed through a receiving space formed inside the screw.

4. The screw surgical device of claim 1, wherein the sensor part monitors the blood flow information by transmitting, by the ultrasound probe, an ultrasound signal to a target object on the insertion path and receiving an ultrasound signal reflected from the target object.

5. The screw surgical device of claim 4, wherein the sensor part monitors the blood flow information using Doppler ultrasound signals.

6. The screw surgical device of claim 1, wherein the information providing part generates alarm information in accordance with the proximity of the ultrasound probe and the vertebral artery on the basis of the blood flow information and provides the alarm information as the guide information.

7. The screw surgical device of claim 1, wherein the information providing part senses that the ultrasound probe comes into contact with the vertebral artery if a blood flow waveform and a blood flow sound included in the blood flow information are outside of a preset normal range.

8. The screw surgical device of claim 1, wherein the information providing part calculates insertion depth information of the screw according to the insertion path on the basis of the blood flow information and provides the insertion depth information as the guide information.

9. A screw surgery method comprising:
inserting an instrument part which includes a gear shift and a taper which are inserted from a target patient's skin to a predetermined point in a pedicle to form an insertion path and a screw which is inserted and fixed into a spine through the insertion path;
monitoring blood flow information about a blood flow passing through an artery adjacent to the insertion path using an ultrasound probe installed at one end of the instrument part; and
sensing a vertebral artery (Vertebral artery, VA) on the basis of the blood flow information and providing guide information about the insertion path.

10. The screw surgery method of claim 9, wherein the gear shift and the taper are installed so that each ultrasound probe is fixed at a terminal of one end in the insertion direction.

11. The screw surgery method of claim 9, wherein the screw is installed so that a separate ultrasound probe is able to be inserted and removed through a receiving space formed therein.

12. The screw surgery method of claim 9, wherein the monitoring blood flow information includes monitoring the blood flow information by transmitting, by the ultrasound probe, a ultrasound signal to a target object on the insertion path and receiving an ultrasound signal reflected from the target object.

13. The screw surgery method of claim 12, wherein the monitoring blood flow information includes monitoring the blood flow information using Doppler ultrasound signals.

14. The screw surgery method of claim 9, wherein the providing guide information includes generating alarm information in accordance with proximity of the ultrasound probe to the vertebral artery on the basis of the blood flow information and providing the guide information.

15. The screw surgery method of claim 9, wherein the providing guide information including sensing that the ultrasound probe comes into contact with the vertebral artery if a blood flow waveform and a blood flow sound included in the blood flow information are outside of a preset normal range.

16. The screw surgery method of claim 9, wherein the providing guide information includes calculating insertion depth information of the screw according to the insertion path on the basis of the blood flow information and providing the insertion depth information as the guide information.
